(19) Europäisches Patentamt
European Patent Office
Office européen des brevets

(11) Publication number: 0 020 788
B1

(12) # EUROPEAN PATENT SPECIFICATION

(45) Date of publication of patent specification: 13.07.83

(51) Int. Cl.³: C 12 Q 1/54, C 12 Q 1/28

(21) Application number: 80900150.6

(22) Date of filing: 04.01.80

(86) International application number: PCT/JP80/00004

(87) International publication number: WO 80/01389 10.07.80 Gazette 80/15

(54) ## TEST PIECE FOR MEASURING GLUCOSE.

(30) Priority: 31.12.78 JP 164968/78

(43) Date of publication of application: 07.01.81 Bulletin 81/1

(45) Publication of the grant of the patent: 13.07.83 Bulletin 83/28

(84) Designated Contracting States: AT DE FR GB LU NL

(56) References cited:
GB - A - 1 159 627
GB - A - 1 395 481
JP - A - 50 120 896
JP - A - 53 016 692
US - A - 3 971 702

CHEMICAL ABSTRACTS, vol. 89, no. 3rd July 1978, page 259, no. 2802z Columbus, Ohio, U.S.A.

(73) Proprietor: KABUSHIKI KAISHA KYOTO DAIICHI KAGAKU
57 Nishiaketa-cho Higashikujo, Minami-ku
Kyoto-shi Kyoto 601 (JP)

(72) Inventor: YAMADA, Shigeki
64-141, Koazafukaya Ohazaterada
Jyoyo-shi, Kyoto 610-01 (JP)
Inventor: YAMAMOTO, Takao
27-11, Shimanouchi-cho Nishinokyo
Nakagyo-ku Kyoto-shi, Kyoto 604 (JP)

(74) Representative: Seaborn, George Stephen et al,
c/o Edward Evans & Co. Chancery House 53-64
Chancery Lane
London WC2A 1SD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England

# Test piece for measuring glucose

This invention relates to a test piece for measuring the concentration of glucose contained in a body fluid such as urine, spinal fluid or blood.

Measurement of the concentration of glucose in body fluids is of importance in clinical examination. It is required particularly to detect glucose in urine and measure its concentration for early detection, diagnosis and control of diabetes and other diseases coexistent therewith.

A test method for measuring the concentration of glucose in body fluids preferably enables measurement to be carried out rapidly and easily without using any instruments and furthermore the test results must have sufficient exactitude to be helpful to diagnosis.

A test piece for glucose has been used heretofore for these purposes. This test piece is made by dipping an absorptive material into a solution containing glucose oxidase, peroxidase, oxidizable chromogen and a buffer agent, and then drying the absorptive material. If the thus produced test piece is dipped into the urine, it becomes coloured to a degree corresponding to the concentration of glucose in the urine as a result of the reactions expressed schematically below. The detection and measurement of glucose become practicable through the examination of this chroma (degree of colouration).

$$\text{glucose} + \text{oxygen} \xrightarrow{\text{glucose oxidase}} \text{hydrogen peroxide} + \text{gluconic acid}$$

$$\text{hydrogen peroxide} + \text{oxidizable chromogen} \xrightarrow{\text{peroxidase}} \text{oxidizing colouring matter} + \text{water}$$

As the oxidizable chromogen, there has been used heretofore a chromogen indicator such as orthotolidine, benzidine or orthodianisidine. However, when detecting or measuring glucose in urine by means of a test piece made with the use of such an indicator, the colour reaction is affected by the specific gravity (the degree of concentration or dilution) of the urine and by any reducing biological substance such as vitamin C (ascorbic acid) present in the urine in addition to the glucose. This is a serious defect because large errors in the measurement are thereby caused. Moreover, these chromogen indicators are carcinogenic and therefore cause a problem for reasons of the safety and health of persons engaged in their production and their use.

In order to eliminate these defects, there is disclosed in JP—A—75—39558 a test piece for approximately measuring glucose concentration. The test piece is coated on its surface with a hydrophobic film-forming polymer, such as cellulose ether and ester, and a water-soluble iodine salt is applied as a chromogen. In order to make this chromogen function effectively as an indicator material, the test piece according to this publication is coated with the hydrophobic film-forming polymer, whereby, so it is stated, the harmful influence of vitamin C (ascorbic acid) can be prevented.

It has been proved, however, in the light of a thorough investigation independently conducted by the present inventors that, as compared with the case where the usual chromogen indicators are used, the test piece disclosed in the above-mentioned patent publication has several defects. Specifically the test piece is 2—3 times slower in reaction time, its detection sensitivity to the presence of glucose in urine is poor (more than 100 mg/dl), and it is wanting in stability to long-term storage due to the lack of stability of the water-soluble iodine salt impregnated therein.

An object of the present invention is to avoid the above-mentioned defects and to provide a test piece for measuring glucose in urine or other body fluid quickly and easily, the measured result being unaffected by the presence of substances other than glucose in the body fluid, its detection sensitivity being sufficient to meet the needs of clinical examination, the test piece having good stability to conservation (storage), and the use of any carcinogenic chromogen being avoidable.

## Disclosure of the Invention

In accordance with the present invention there is provided a test piece for the determination of glucose in body fluid, said piece having been made by impregnating or painting a substrate with a solution of an enzymatic species displaying the activity of glucose oxidase, a substance having peroxidase-like activity, oxidizable chromogen, and a buffering agent; characterized in that said oxidizable chromogen is a mixture of guaicum resin (also known as guaiac, guaiac resin or guaiac gum) or an active constituent thereof, and N,N′-tetramethyl-4,4′-diaminodiphenylmethane or N,N′-tetramethyl-3,3′-diaminodiphenylmethane.

We refer below to N,N′-tetramethyl-4,4′-diaminodiphenylmethane or N,N′-tetramethyl-3,3′-diaminodiphenylmethane as tetrabase.

Preferably the solution contains polyvinylbutyral so that any possible adverse effect of vitamin C (ascorbic acid) on the measurement of glucose is reduced.

Brief Description of Drawings

Figure 1 is a graph or standard curve showing the relationship between the concentration of glucose in urine and the reflectivity of test pieces which have been dipped into the urine, the test pieces having been made using guaicam resin only as an oxidizable chromogen;

Figure 2 is a graph of a similar standard curve relating to test pieces made using tetrabase only as an oxidizable chromogen; and

Figure 3 is a graph of a standard curve relating to test pieces prepared as described below in Example 1 of this invention.

Best Mode for Carrying out the Invention

In order to explain the invention in detail reference is made below to examples and to the accompanying drawings.

The test piece of the invention is made by impregnating or painting a substrate with an enzymatic species displaying glucose oxidase activity, a substance having peroxidase-like activity, a buffer agent, and guaicum resin or an active constituent thereof and tetrabase as a chromogen, and optionally further with polyvinylbutyral. When thus produced test piece is dipped into body fluid which is to be examined, or the latter is painted on the former, the test piece displays a chroma correspondingly to the concentration of glucose in the body fluid. The test piece of the invention, as such, is not influenced by the composition of the examined body fluid (except its glucose content), does not contain any carcinogenic substance, has excellent detection sensitivity and excellent stability to conservation (storage), and thereby overcomes various defects of test pieces made by conventional techniques.

The principle of colouration of the test piece of the invention will be given schematically below. Glucose oxidase oxidizes specifically glucose to produce hydrogen peroxide and gluconic acid in a quantity corresponding to the concentration of glucose. Then this hydrogen peroxide oxidizes guaicum resin and tetrabase by the action of peroxidase to form oxidizing colouring matter and, as a result, presents the concentration of glucose. By observing the chroma with the naked eye or by means of a light reflectometer, it becomes possible to detect and measure the glucose in the examined body fluid.

$$\text{glucose} + \text{oxygen} \xrightarrow{\text{glucose oxidase}} \text{hydrogen peroxide} + \text{gluconic acid}$$

$$\text{hydrogen peroxide (guaicum resin and tetrabase)} \xrightarrow{\text{peroxidase}} \text{oxidizing colouring matter} + \text{water}$$

Conventionally in the prior art there have been used chromogen indicators such as orthotolidine, benzidine and orthodianisidine. These kinds of indicators have a high colour sensitivity, so that they are capable of detecting the presence of glucose to the full in the presence of a small quantity of hydrogen peroxide generated by the action of glucose oxidase and also in the presence of a small quantity of peroxide, and moreover they can present a chroma corresponding to the concentration of glucose. However, if some reducing biological substances, such as vitamin C, should be present in the body fluid, for example urine, which is to be examined, they will consume the small quantity of hydrogen peroxide produced by the action of glucose oxidase, so that there may occur no colouration or, if any, a chroma lower than normal, which will lead to serious error in measurement. Moreover, in urine is contained uric acid resulting from metabolism. Since this uric acid is also a reducing substance, it presents a chroma lower than normal in the case of concentrated urine in which the specific gravity is larger than usual, while on the other hand it presents a higher chroma in the case of dilute urine. This leads likewise to serious error in measurement.

The present inventors found that when glucose oxidase is present on the test pieces made by conventional techniques in a quantity necessary for the colour reaction, the oxidative activity of the glucose oxidase is affected by the concentration of sodium chloride contained in the urine, that in the concentration range of sodium chloride contained in normal urine the concentration of sodium chloride is inversely proportional to the activity of glucose oxidase, and that if glucose oxidase is used in a large quantity, the influence of the concentration of sodium chloride can be reduced.

The present inventors also found that the hydrogen peroxide produced by the action of glucose oxidase is consumed by reducing substances, in consequence of which measurement errors take place, that in order to prevent such errors, it is necessary to produce hydrogen peroxide in a greater quantity, whereby the proportion of it consumed is reduced, and that having regard to the above-mentioned correlation between the concentration of sodium chloride and the activity of glucose oxidase, glucose oxidase should be applied in a large quantity to the test piece.

However, as previously stated, chromogen indicators such as orthotolidine, benzidine and orthodianisidine have a good colouration sensitivity and accordingly, if glucose oxidase is used in a large quantity, they colour in response to the glucose more than required, so that the range of measurements becomes narrow. It follows from this that test pieces with such chromogen indicators are unsuitable for clinical examination. Thereupon, the inventors considered using another chromogen having lower colouration sensitivity.

Then the inventors, who had been seeking an oxidizable chromogen having a colouration sensitivity of a low to moderate degree and, if possible, being non-carcinogenic, finally thought of guaicum resin. When they applied this resin, in accordance with the aforesaid purpose, to the test piece and tested the thus produced test piece by using a urine sample which contained glucose of a known concentration, then it presented a practically constant chroma independently of the concentration of glucose at more than about 200 mg/dl of the latter. The standard curve graphing the correlation between the reflectivity and the concentration of glucose in the urine was constructed with the use of a light reflectometer having a measuring wavelength of 620 nm corresponding to the hue generated from the oxidized guaicum resin. The standard curve was as shown in Figure 1. Then the inventors thought that it might be better if an oxidizable chromogen having a colouration sensitivity a little lower than guaicum resin and also having an oxidation-caused hue of the same degree as the hue of guaicum resin were used in combination with guaicum resin. They searched for such kind of oxidizable chromogen, until they recognized that the compounds we refer to as tetrabase are such substances. There is graphed in Figure 2 the standard curve showing the correlation between the reflectivity and the concentration of glucose in the urine on a test piece made by using N,N'-tetramethyl-4,4'-diaminodiphenylmethane only as an oxidizable chromogen. In the case of the test piece made by using this tetrabase only, the colouration starts from a concentration of glucose of about 200 mg/dl and the chroma varies continuously in the range of 200 up to at least 1000 mg/dl.

From the above-mentioned, the inventors guessed that the object of this invention would be achieved by using a mixture of guaicum resin and tetrabase as oxidizable chromogen. The results of experiments turned out even as they guessed. It was shown that the mixing ratio of both is practicable at 1:1 to 1:10 and the optimum ratio is from 1:2 to 1:4. For example, there is shown in Figure 3 the standard curve graphing the correlation between the reflectivity and the concentration of glucose in urine on a test piece, in which the mixing ratio is 1:3. In the case of this test piece, the measurement can be made when the concentration of glucose in the urine is of 0 to 1000 mg/dl, enabling the glucose to be detected at the sensitivity necessary for clinical examination.

In this way, if guaicum resin and tetrabase are used jointly as oxidizable chromogen, and glucose oxidase is contained in the test piece in a large quantity, they are scarcely affected by variations in the specific gravity of the examined urine taken from the patient under normal conditions or by the presence of reducing substances such as vitamin C or glutathione present in the urine as biological compounds, thereby making possible the accurate measurement of glucose. For all that, there is a very rare special case wherein at least 50 to 100 mg/dl of vitamin C is present in the examined urine of the patient directly after the inoculation of a large quantity of vitamin C.

The inventors researched further for the purpose of finding how to cope with the presence of a large quantity of vitamin C in such a very rare special case. As a result they found that it was possible substantially to completely eliminate the effect of vitamin C by making the test piece according to the invention contain polyvinylbutyral. The application of organic synthetic polymers such as polyvinylpyrrolidone, polyvinyl alcohol and ethyl cellulose, all having the same character as the above polyvinylbutyral, likewise to the test piece of our invention exhibited no effect. It was only polyvinylbutyral that displayed a marked effect. Though it is unclear why it is so, it is envisaged that probably, due to the polyvinylbutyral, reductive substances such as vitamin C are held on the surface of the test piece, so that their participation in the colour reaction might be suppressed.

Thus the present invention utilizes a mixture of guaicum resin and tetrabase as an oxidizable chromogen and preferably polyvinylbutyral.

The test piece of the invention for use in the measurement of glucose contains an enzymatic species displaying glucose oxidase activity, a substance having peroxidase-like activity, guaicum resin, tetrabase, buffering agent and, preferably, polyvinylbutyral. The enzymatic species displaying glucose oxidase activity may be any substance having an enzymatic action capable of generating hydrogen peroxide by specifically oxidizing glucose. The substances having peroxidase-like activity may be any substance having an enzymatic activity capable of oxidizing both guaicum resin and tetrabase with the help of hydrogen peroxide. The buffering agent may be any substance which maintains a suitable pH in the range of 3.0 to 7.5. Any polyvinylbutyral having a degree of polymerization in the range of 200 to 1500 is suitable. The polyvinylbutyral may be an industrial product which is a copolymer of vinylbutyral, vinyl acetate and vinyl alcohol. The guaicum resin may be natural guaic resin.

Instead of guaicum resin, any of guaiaconic acid, guaiarethene acid and guaiac acid which are the main constituents of guaicum resin, may be used. As mentioned above, the tetrabase may be N,N'-tetramethyl-4,4'-diaminodiphenylmethane or N,N'-tetramethyl-3,3'-diaminodiphenylmethane. The test piece may furthermore incorporate one or more additives such as, for example, a protective agent, a concentrating agent, a wetting agent, a surface active agent and an inert dyestuff for giving some ground colour.

In order to produce the test piece of our invention, initially a solution is made, which contains glucose oxidase, peroxidase, and the buffering agent of the afore-mentioned composition. Into this solution is dipped an absorptive material made of a filter paper or the like. Then the absorptive material is taken out therefrom and is left to dry. Then the absorptive material is dipped into a solution of guaicum resin, tetrabase and polyvinylbutyral and left to dry. It is possible to use instead of the above-mentioned absorptive substrate a film, made for example of polyvinyl chloride, as the substrate. In this case, a solution containing the aforesaid components is prepared by mixing therewith adhesive which aids the adhesion of the above composition to the film, silica gel which promotes the permeation of the body fluid to be examined thereinto, and porous particles such as titanium oxide granules. After this solution has been coated or painted onto the film, it is left to dry, in order to obtain the test piece.

When the test piece thus produced for the measurement of glucose is dipped into the body fluid which is to be examined or the latter is painted on the former, there is exhibited the chroma correspondent to the concentration of glucose. If a standard simple colorimetric table compiled beforehand corresponding to the actual variations of the concentration of glucose is prepared, it is possible to measure approximately and easily the glucose by comparing the coloured chroma observed with the naked eye with the above standard colorimetric table. Furthermore, if there have been prepared different standard curves showing the correlations between the reflectivities and the concentrations of glucose based on the results of the measurements of the reflectivities of the test pieces by means of a reflectometer having measuring light wavelengths correspondent to the coloured hues, while using standard solutions having the various concentrations of glucose, it is also possible to estimate the glucose.

The invention is illustrated by the following examples.

Example 1

800 mg of sodium alginate (1000 mPas), 3.3 g of citric acid · $H_2O$, and 8.7 g of citric acid-3-sodium · 2-$H_2O$ were added to 100 ml of purified water and dissolved by stirring, whereto was further added 600 mg of glucose oxidase (110 U/mg), and 100 mg of peroxidase (100 U/mg), which was likewise dissolved by slowly stirring. In such a manner was made the solution for the first stage treatment. After a filter paper for chromatographing had been dipped into and saturated with this solution, it was pulled out therefrom to be left to dry at 50°C for 1.5 hours.

Then, 0.5 g of guaicum resin and 1.5 g of N,N'-tetramethyl-4,4'-diaminodiphenylmethane were added to 100 ml of acetone and were dissolved to obtain the solution for the second stage treatment. Into this solution was dipped the filter paper which had finished the first stage treatment and the filter paper was then left to dry at 45°C for 30 minutes. The filter paper thus treated constituted a test piece according to the invention.

The test piece prepared in this way was cut into bits. Each bit was stuck onto one end of a rectangular strip-shaped plastics' film by the use of a double-faced adhesive tape. These bits were kept in a hermetically sealed receptacle containing silica gel as a dessicant, thereby being able to be conserved or stored stably for more than a year. They could be conveniently taken out from the receptacle and used whenever needed.

When this piece was pulled up directly it had been dipped into examined urine, it coloured blue. The chroma corresponded to the concentration of glucose in the urine, so that the approximate measurement of glucose was feasible through its colour comparison by the naked eye with reference to a previously prepared standard colorimetric table.

Aside from this, it was possible to measure the glucose by means of a standard curve (Figure 3) showing the correlation between both the reflectivity measured at the time of the colouration of the test piece through the use of a reflectometer which irradiated the surface of the test piece with monochromatic light of 620 nm and measured the reflected light from the test piece, and the concentration of glucose, which standard curve had been worked out in advance by the use of standard solutions of glucose of various known concentrations.

Example 2

To a buffering solution which has been made in advance by adding 4.7 g of potassium phosphate ($K_2HPO_4$) and 3.8 g of sodium phosphate ($Na\ H_2PO_4H_2O$) to 100 ml of purified water and by stirring them, 1.0 g of gelatine was added, stirred thoroughly, and dissolved, whereto 800 mg of glucose oxidase (110 U/mg) and 105 mg of peroxidase (130 U/mg) were added, stirred slowly, and dissolved. In this manner was prepared the solution for the first stage treatment. Into this solution was dipped the qualitative filter paper, which was then pulled out and left to dry at 45°C for two hours.

The solution for the second stage treatment was made by adding 1.0 g of guaicum resin, 1.5 g of N,N' - tetramethyl - 4,4' - diaminodiphenylmethane, 0.5 g of TWEEN 20 (trade name of an interfacial activator), and 2.5 g of polyvinylbutyral (having a degree of polymerization of 700) to a mixed solvent of acetone-toluene-ethyl alcohol in the ratio of 1:1:2 and by dissolving them in the solvent. Into this solution was dipped the filter paper after having been subjected twice to the first stage treatment. The filter paper was then dried at 45°C for 60 minutes. The filter paper thus treated constituted a test piece according to the invention.

The conservability or storability and manner of use of this test piece were the same as those of the test piece in Example 1.

In the above, reference has been made to some preferred examples for working of the present invention. However, modification and variation is possible in the substances constituting the test piece: For example, any enzyme can be applied regardless of its origin provided it displays glucose oxidase activity. In the same way, as substances having the peroxidase-like activity, hemoglobin, urohemin, and the like can be substituted for peroxidase. As a buffering agent can be used, along with those originating from citric acid and phosphoric acid, a mixture of citrate and phosphate, tartrate, amino acid salt or the like, as long as they are substances having the effect of maintaining the pH value in the range of pH 3.0 to 7.5.

In the place of guaicum resin which is available as a natural substance under normal conditions, it is possible to use guaiaconic acid, guaiarethane acid, or guaiac acid, these being the principal constituents of guaicum resin. It is further possible to employ carboxymethylcellulose, soluble starch, cow-serum albumin or the like as protective agent or concentrating agent in place of gelatine and sodium alginate; polyethylene glycol and polyvinylpyrrolidone as wetting agent; and cationic or anionic surface active agent or the like as surface active agent beside non-ionized surface active agents such as "TWEEN 20" mentioned above. For the inert dyestuff giving a ground colour, the food colouring matter; yellow 4, for example, can be compounded if desired. With regard to the material of the test piece, the working of the invention can be conducted, separately from the previously mentioned absorptive material, such as made of filter paper, cloth, or wood piece, by painting some suitable adhesive or porous particles directly onto a material such as a film, for example, made of polyvinyl chloride or polyester to form the substrate of the test piece.

To examine the effect of this invention, we made an investigation by comparison on a test piece for measuring glucose made conventionally using orthotolidine as chromogen, on one hand and on the test pieces according to the invention described in Examples 1 and 2 above, on the other, in relation to the specific gravity of the urine, and to the influence of vitamin C present with glucose in the urine. The results were as follows:

As a start, four kinds of urine (A, B, C, and D) having different specific gravities were collected and two sorts of urine samples were made by adding glucose to the four kinds of urine to adjust their glucose contents to 100 mg/dl and 400 mg/dl, respectively. The urine samples were then tested with the aforementioned test pieces. The respective reflectivities of the test pieces were measured with the reflectometer using monochromatic light of wavelength 620 nm corresponding to the coloration of the test pieces. In this test, the reaction time was set to be 40 seconds. As shown from the results in Table 1, the test piece made by conventional technique was seriously affected by the specific gravity of the urine, while on the contrary, the test pieces according to Example 1 and 2 of the invention was scarcely affected by the specific gravity of the urine.

TABLE 1

| Concentration of glucose of sample | Test object | A | B | C | D |
|---|---|---|---|---|---|
| | Specific gravity | 1.007 | 1.019 | 1.025 | 1.043 |
| 100 mg/dl | Conventional manufacture | 21.3 | 55.5 | 66.4 | 87.2 |
| | Example 1 | 55.2 | 55.7 | 57/1 | 58.6 |
| | Example 2 | 56.6 | 59.8 | 58.0 | 57.3 |
| 400 mg/dl | Conventional manufacture | 16.9 | 22.4 | 53.8 | 75.2 |
| | Example 1 | 24.3 | 26.9 | 27.1 | 29.2 |
| | Example 2 | 31.5 | 31.3 | 33.3 | 34.7 |

Note: Numerical values in the table show the reflectivities.

The next test was conducted in like method with the use of the two sorts of urine samples prepared by adding glucose to the urine of an average specific gravity so that their resulting glucose contents were 100 mg/dl and 400 mg/dl, and furthermore by adding vitamin C to the respective urine samples to provide concentrations of vitamin C of 10, 30, 50, and 100 mg/dl. The results of measuring reflectivities of the test pieces are given in Table 2 in the same way as in the former test. From this Table, it can be seen that the test piece made conventionally, was seriously affected by the presence of vitamin C, while the test piece of Example 1 of this invention was scarcely affected by the results of vitamin C in an amount of up to 50 mg/dl, and the test piece of Example 2 was hardly affected by the presence of vitamin C in an amount of up to 100 mg/dl.

TABLE 2

| Concentration of glucose of sample | Concentration of vitamin C of sample | 0 | 100 mg/dl | 30 mg/dl | 50 mg/dl | 100 mg/dl |
|---|---|---|---|---|---|---|
| 100 mg/gl | Conventional manufacture | 55.5 | 62.6 | 76.5 | 89.6 | 93.1 |
| | Example 1 | 55.7 | 56.0 | 76.5 | 89.6 | 76.1 |
| | Example 2 | 59.8 | 58.6 | 59.1 | 61.2 | 62.3 |
| 400 mg/dl | Conventional manufacture | 22.4 | 34.41 | 39.1 | 59.6 | 89.3 |
| | Example 1 | 26.9 | 26.5 | 29.1 | 28.5 | 49.6 |
| | Example 2 | 31.3 | 30.0 | 32.4 | 32.9 | 36.8 |

Note: Numerical values in the table show reflectivities.

Industrial Applicability

As described particularly in the above, the present invention provides a test piece for the measurement of glucose that provides the measured result free from the adverse influence of the composition of the examined body fluid, that has excellent detection sensitivity and also excellent in the stability of conservation, and that does not use any carcinogenic chromogen, thereby enabling the measurement of glucose in the body fluid to be conducted rapidly and simply with the satisfactory exactitude so far as clinical examination is concerned. The test piece of our invention has a very high practical value in examining and controlling diabetes and other diseases co-existent therewith.

**Claims**

1. A test piece for the determination of glucose in body fluid, said piece having been made by impregnating or painting a substrate with a solution of an enzymatic species displaying the activity of glucose oxidase, a substance having peroxidase-like activity, oxidizable chromogen, and a buffering agent; characterized in that said oxidizable chromogen is a mixture of guaicum resin or an active constituent thereof and N,N'-tetramethyl-4,4'-diaminodiphenylmethane or N,N'-tetramethyl-3,3'-diaminodiphenylmethane.

2. A test piece as claimed in Claim 1, wherein said solution contains polyvinylbutyral.

3. A test piece according to Claim 2, wherein the polyvinylbutyral has a degree of polymerisation of 200 to 1500.

4. A test piece according to any preceding claim, wherein the oxidizable chromogen comprises guaicum resin or guaiaconic acid, guiarethene acid or guaiac acid.

5. A test piece according to any preceding claim, wherein the guaicum resin or the active constituent thereof and the N,N'-tetramethyl-4,4'-diaminodiphenylmethane or N,N'-tetramethyl-3,3'-diaminodiphenylmethane are present in the ratio by weight of 1:1 to 1:10.

6. A test piece according to Claim 4, wherein said ratio is 1:2 to 1:4.

**Revendications**

1. Un toucheau pour la détermination du glucose dans un liquide physiologique, ce toucheau ayant été préparé par imprégnation ou revêtement d'un support à l'aide d'une solution d'une espèce enzymatique présentant l'activité de la glucose-oxydase, d'une substance ayant une activité analogue à celle de la peroxydase, d'un chromogène oxydable et d'un agent tampon, caractérisé en ce que le chromogène oxydable consiste en un mélange de résine de gaïac ou d'un constituant actif de cette résine et de N,N' - tétraméthyl - 4,4' - diaminodiphénylméthane ou de N,N'-tétraméthyl-3,3'-diaminodiphénylméthane.

2. Un toucheau selon la revendication 1, dans lequel ladite solution contient du polyvinylbutyral.

3. Un toucheau selon la revendication 2, dans lequel le polyvinylbutyral est à un degré de polymérisation de 200 à 1.500.

4. Un toucheau selon l'une quelconque des revendications qui précèdent, dans lequel le chromogène oxydable comprend de la résine de gaïac ou de l'acide gaïaconique, de l'acide gaïaréthénoïque ou de l'acide gaïacique.

5. Un toucheau selon l'une quelconque des revendications qui précèdent, dans lequel la résine de gaïac ou son constituant actif de la N,N' - tétraméthyl - 4,4' - diaminodiphénylméthane ou la N,N'-tétraméthyl-3,3'-diaminodiphénylméthane sont présents dans des proportions relatives en poids de 1:1 à 1:10.

6. Un toucheau selon la revendication 4, dans lequel ledit rapport va de 1:2 à 1:4.

**Patentansprüche**

1. Teststück für die Bestimmung von Glukose in Körperflüssigkeit, wobei das Stück durch Imprägnieren oder Bestreichen eines Substrats mit einer Lösung einer enzymatischen Spezies, welche die Aktivität von Glukose-Oxidase entfaltet, einer Substanz mit Peroxidase-ähnlicher Aktivität, oxidierbarem Chromogen und einem Puffermittel, hergestellt worden ist, dadurch gekennzeichnet, daß das oxidierbare Chromogen ein Gemisch von Guaiacumharz oder eines aktiven Bestandteils davon und N,N'-Tetramethyl-4,4'-diaminodiphenylmethan oder N,N' - Tetramethyl - 3,3' - diaminodiphenylmethan ist.

2. Teststück nach Anspruch 1, worin die Lösung Polyvinylbutyral enthält.

3. Teststück nach Anspruch 2, worin das Polyvinylbutyral einen Polymerisationsgrad von 200 bis 1500 aufweist.

4. Teststück nach einem der voranstehenden Ansprüche, worin das oxidierbare Chromogen Guaiacumharz oder Guaiaconsäure, Guaiarethensäure oder Guaiasäure enthält.

5. Teststück nach einem der voranstehenden Ansprüche, worin das Guaiacumharz oder der aktive Bestandteil davon und das N,N'-Tetramethyl-4,4'-diaminodiphenylmethan oder N,N' - Tetramethyl - 3,3' - diaminodiphenylmethan in einem Gewichtsverhältnis von 1:1 bis 1:10 vorhanden sind.

6. Teststück nach Anspruch 4, worin das Verhältnis 1:2 bis 1:4 ist.

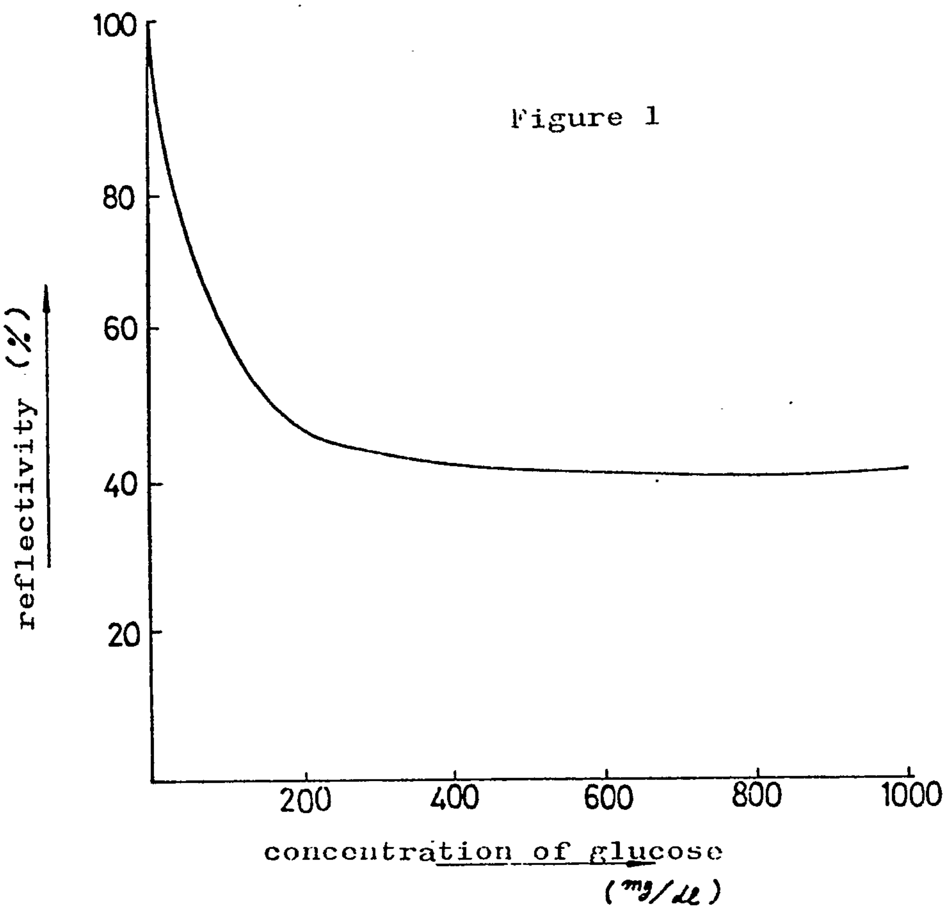
Figure 1
100
80
60
40
20
reflectivity (%)
200
400
600
800
1000
concentration of glucose
(mg/dl)

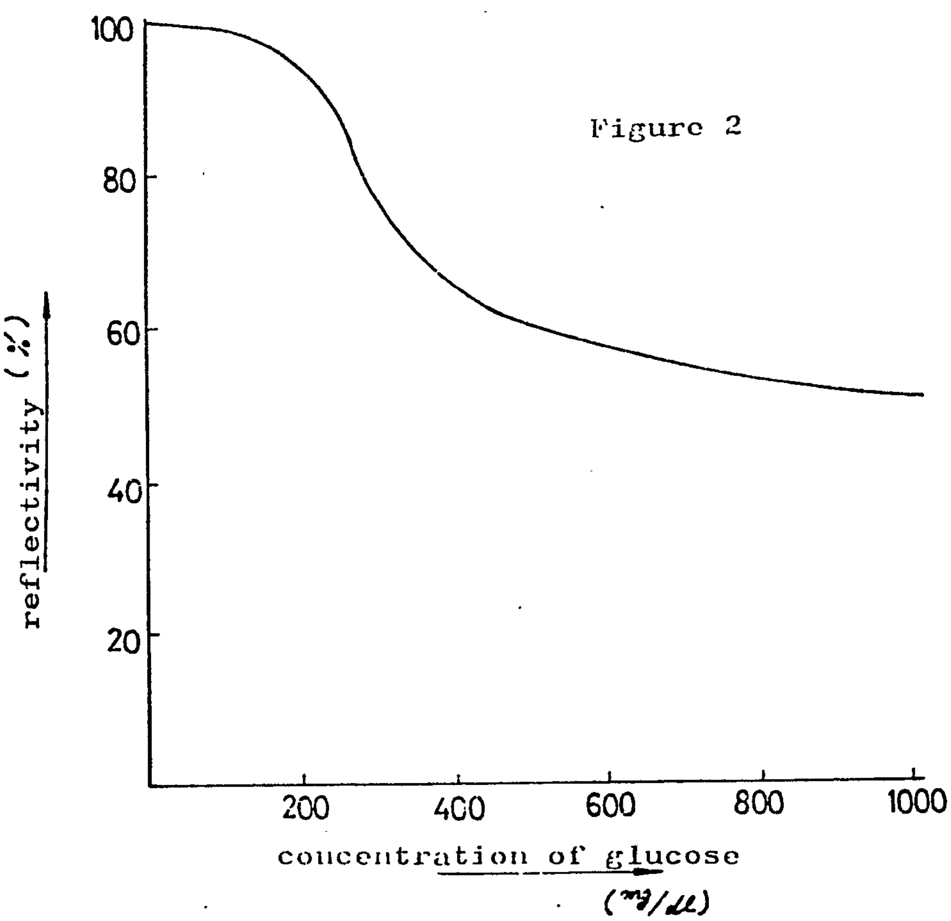
Figure 2
100
80
60
40
20
reflectivity (%)
200
400
600
800
1000
concentration of glucose
(mg/dl)

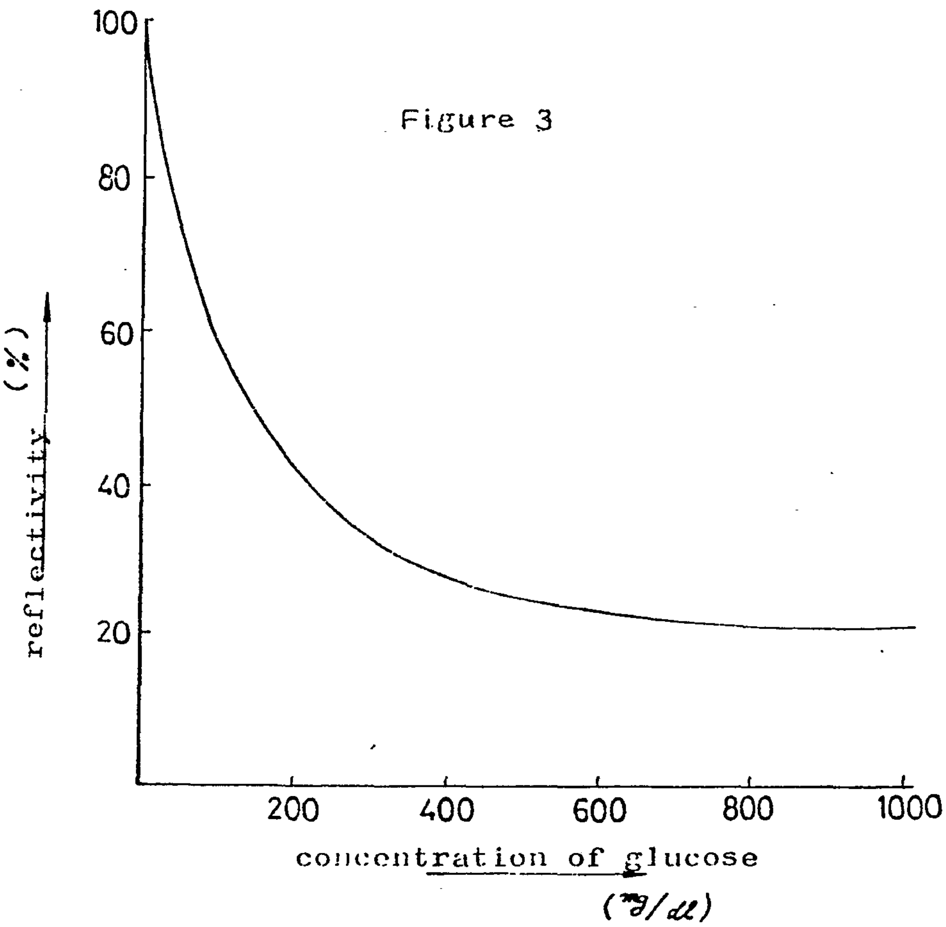
Figure 3
100
80
60
40
20
reflectivity (%)
200
400
600
800
1000
concentration of glucose
(mg/dl)